# EUROPEAN PATENT SPECIFICATION

(11) **EP 3 052 144 B1**
(45) Date of publication and mention of the grant of the patent: **16.11.2022**
(21) Application number: 13774389.4
(22) Date of filing: 03.10.2013
(51) Int. Cl.: A61L 2/24

(54) **SYSTEM FOR WASHING, DISINFECTING AND/OR STERILIZING MEDICAL, DENTAL, LABORATORY AND/OR PHARMACEUTICAL GOODS**
SYSTEM ZUM WASCHEN, DESINFIZIEREN UND/ODER STERILISIEREN VON MEDIZIN-, ZAHNMEDIZIN-, LABOR- ODER PHARMAZIEPRODUKTEN
SYSTÈME POUR LAVER, DÉSINFECTER ET/OU STÉRILISER DES PRODUITS MÉDICAUX, DENTAIRES, DE LABORATOIRE ET/OU PHARMACEUTIQUES

(43) Date of publication of application: 10.08.2016
(73) Proprietor: Getinge Sterilization AB, 305 05 Getinge (SE)
(72) Inventor: PETERSSON, Anna, 432 94 Varberg (SE); NORLIN, Per, 423 53 Torslanda (SE)
(74) Representative: Valea AB
(86) International application number: PCT/EP2013/070627
(87) International publication number: WO 2015/049001

(56) References cited:
- WO-A1-86/04698
- WO-A1-2005/004931
- WO-A2-2007/021696
- WO-A2-2007/126883
- US-A1- 2009 062 610

## Description

### TECHNICAL FIELD

The present disclosure relates to a system comprising a device for washing, disinfecting and/or sterilizing medical, dental, laboratory and/or pharmaceutical goods. The disclosure further relates to a process for supply of a medium to the system.

### BACKGROUND

The pharmaceutical industry, hospitals, care centers, dental care centers, laboratories and similar industries and facilities are constantly struggling against contaminations such as bacterial infections and viral infections. Hygienic issues are constantly on the agenda and continuously evaluated. One hygienic issue of special significance is the washing, disinfecting and sterilization of reusable and consumable goods, as for example being performed in a central sterile services department, CSSD, of a hospital. Reusable goods can be surgical equipment, such as knives, graspers, clamps, retractors, dilators, probes, scopes, drills, and saws, laboratory goods, such as bottles, bowls, condensers, funnels, flasks, pipettes and plates, or the like. Examples of consumable goods are paper or broth. Any item of medical, dental, laboratory and/or pharmaceutical goods and/or equipment, which is intended to be used, and which can be contaminated with hazardous or biological substances, is the subject of strict hygienic conditions and needs to be washed, disinfected and/or sterilized. Further, bio-hazardous material is in some cases sterilized before being disposed of.

The process of washing, disinfecting or sterilizing of medical, dental, laboratory and/or pharmaceutical goods, such as the goods mentioned above, is a demanding process in terms of the facilities used, the staff, the process parameters, the apparatuses and even the ambient environment surrounding the apparatuses. All restrictions and conditions serving the purpose to reduce or eliminate the risk for contamination make it difficult and costly to operate washing, disinfecting and/or sterilization processes.

For example, a pharmaceutical production site may be equipped with steam generators, water pretreatment apparatuses, closure processing systems (CPS), closure processing discharge systems, component washers, glassware washers, terminal sterilization systems, isolators and sterility testing equipment, simply to wash, disinfect and/or sterilize different medical or laboratory goods.

Sterilization, also spelt sterilisation, herein relates to a process that eliminates, i.e. kills, microbial life, including transmissible agents, such as fungi, bacteria, viruses, spore forms, nucleic acid etc., present on or in a surface, contained in a fluid, a powder or a solid, in medication, and/or on and/or in a compound such as biological culture media or any other solution and/or formula. Sterilization can be achieved by applying heat, chemicals, radiation, high pressure, and filtration or combinations thereof.

Steam sterilization, or autoclaving, involves subjecting goods to steam at a high temperature. Steam sterilization involves the use of saturated steam under pressure and is a non-toxic method for sterilization. Further, steam sterilizers, or autoclaves are available in different sizes for different purposes.

Four factors are relevant for the outcome of steam sterilization: steam, pressure, temperature and time. Steam sterilization procedures require sterilization at high temperatures, see for example "Guideline for Disinfection and Sterilization in Healthcare Facilities, 2008", US Centers for Disease Control and Prevention, and "Bundesgesundheitsbl-Gesundheitsforsch-Gesundheitsschutz 11.2001" Robert Koch Institut, Germany, such as above 100°C, 110°C, 120°C or 130°C.

A device for sterilizing medical, dental, laboratory and/or pharmaceutical goods should preferably be adapted to operate at a temperature above 100 °C, preferably above 120°C, more preferably between 120-140°C and at a suitable pressure, typically but not limited to at least above 101,3 kPa. A sterilizer disclosed herein should preferably at least meet the requirement of EN285:2006 and A2:2009, standards used in this field of technology and known to the skilled person in the art.

The sterilization time required varies depending on the goods to be sterilized and the temperature used. Chemical and biological indicators are available for monitoring the sterilization process and to ensure that sterility is achieved. Properly executed steam sterilization will inactivate all fungi, bacteria, viruses and bacterial spores. If not all fungi, bacteria, viruses and bacterial spores can be removed or inactivated, the temperature, time and pressure are selected so that the sterilization device, and the method, has a Sterility Assurance Level, SAL, of typically at most 1/ 1.000.000, preferably lower than 1/1.000.000. SAL is used to describe the probability of a non-sterile goods exiting the device or method after the sterilization process has been completed.

Another example of a process for washing, disinfecting and/or sterilizing is high level disinfection (HLD), which is an accepted standard for the reprocessing of semi-critical devices, including flexible endoscopes, or for sterilization of critical or semi-critical devices that are heat-sensitive or incompatible with traditional sterilization methods. Endoscope reprocessing, for instance, involves the cleaning and disinfection of endoscopes, and may encompass the steps of cleaning, rinsing, disinfection, secondary rinsing, drying and storing. For drying, a drying cabinet is commonly utilized, which drying cabinet may comprise a door, a cabinet, a control circuit and a loading system such as one or several shelves or hooks. Use of the drying cabinet enables for immediate reuse of e.g. endoscopes, even after extended storage periods thereof.

To ensure a correct process performed by devices used for washing, disinfecting and/or sterilizing in such high-demanding applications, for example, but not limited to, sterilizers, washers, autoclaves and washer-disinfectors, a certain quality of incoming media, such as water, steam and/or a chemical, is desirable. The media are used for performing the process of washing, disinfecting and/or sterilizing in the device. A medium may be used constantly throughout the time the process is performed, , or it may be used intermittently during the process, e.g. water. There are also media which are only used for one or more certain processes in the device, e.g. a chemical. A medium may be reused in different process steps.

Today, parameters for the different media may be monitored and controlled by the different devices supplying the incoming media. Each of these parameters is usually controlled separately and not interconnected, or not controlled or measured at all.

It is desirable, and sometimes essential and/or even required, that the incoming medium has an acceptable quality. Otherwise the device may break down and/or there is a risk of insufficient processing of the goods, e.g. insufficiency in sterilization.

Today, it can be a very challenging, time-consuming and costly task, to investigate the cause of such problems, since many different devices have to be checked, and these devices may be located at different physical locations. Since it is often believed that the problems are caused by a defective device, a technician may be called to maintain or repair the device. However, if the problems are due to poor quality of an incoming medium, the technician will not find any defects in the device. It can then be very difficult to afterwards establish that the problem was due to poor quality of an incoming medium, e.g. due to fluctuations in water quality. Further, and especially if the monitoring systems of the incoming media are located in other physical locations than the device, it will take time and involve extra work to check that the supply systems for incoming media work as intended.

WO 2007/126883 A2 relates to sterilization methods and systems that may be used within numerous contexts, such as health-care and manufacturing facilities.

US 2009/0062610 A1 relates to an endoscope reprocessor having a water supply disinfection filter and a method for self-disinfection of the filter employing a pair of connectors to switch from a normal operating mode into a self-disinfection mode in which circulating germicidal fluid within the reprocessor flows through the filter, while the water supply remains connected to the system and isolated from the circulating fluid.

US 2011/076192 A1 relates to a method and an apparatus for sterilizing an article by sequentially exposing the article to hydrogen peroxide and ozone.

### SUMMARY

The object of the present disclosure is to overcome and/or ameliorate at least one of the disadvantages of the prior art, or to provide a useful alternative.

The present disclosure provides a system having an improved possibility to monitor, and optionally control, the quality of incoming media.

A system is provided, which is easy to handle, and which meets high hygienic demands. Thus, in a first aspect of the present invention there is provided a system according to claim 1.

The system comprises
- a device for washing, disinfecting and/or sterilizing medical, dental, laboratory and/or pharmaceutical goods, and
- a parameter determining unit adapted to determine a value indicative of a parameter of a medium dedicated to the device before or when being supplied to the device.

Thus, only the amount or quantity of medium which is intended to be supplied to the device is monitored by the parameter determining unit.

The device may relate to a device used in the pharmaceutical industry, hospitals, care centers, dental care centers, laboratories and similar industries and facilities mentioned above, having in common that they have high hygienic demands. The present disclosure does not relate to the kind of dishwashers or washing machines used in homes, hotels, restaurants or catering, which have a lower hygienic demand than the above-mentioned apparatuses, even if the device of the present disclosure would be possible in such places, in case high hygienic demands are desired.

The medium supplied to the device is used for a process performed in the device, e.g. washing, disinfecting and/or sterilizing. The process may e.g. comprise high level disinfection or re-processing e.g. of endoscopes. The process may be continuous or may be performed batch-wise. Examples of different media are water, steam, a solvent or a chemical, such as detergent. A medium may be used all the time the process is performed, or it may be used intermittently during the process, e.g. water. There are also media which are only used for certain processes in the device, e.g. a chemical. The medium may be supplied as a fluid, e.g. being fed through a conduit. The medium may be a liquid, a powder, a crystal, a solid, a semi-solid, a gas or an aerosol. The medium may be supplied continuously, intermittently or batch-wise. The electricity, not included in the media of the claims may be supplied by the general electricity network or be supplied by special energy sources, such as an emergency power system, in case of a failure of the general electricity network. The medium may be a consumable, such as a solvent, a detergent or a chemical. Not included in the claims, the medium may also be a radiation, such as UV-light radiation or x-ray radiation.

The device may also comprise means for packaging of the medical, dental, laboratory and/or pharmaceutical goods. In that case, the process may, if desired, in addition to the washing, disinfecting and/or sterilizing, also comprise a step of packaging the goods. The packaging will help to maintain the treated goods washed, disinfected and/or sterilized after the process has been performed. As an alternative, or as a complement, the goods may be manually packed, if desired.

For each medium, one or more parameters may be determined and possibly monitored. The type of parameter depends on the type of medium. The parameter is a quality parameter, i.e. a parameter representative of a quality aspect of the medium. The quality parameter may be a parameter which is essential for optimum or even acceptable performance of the device and/or for performing the process therein. Often, the quality parameter should fulfil a certain qualitative standard for achieving a guaranteed performance of the device or process performed therein. Examples of quality parameters are temperature, pH, conductivity, charge, density, pressure. If the determined parameter is temperature, one or more other parameters may be determined in addition.

The parameter determining unit may determine one or more parameters of the medium. As an alternative, or in addition, there may be more than one parameter determining unit for the medium, determining different parameters of the medium, or the same.

Depending on the type of parameter, it may be determined directly or indirectly, such that it is possible to determine a value indicative of the parameter. The parameter may e.g. be measured by one or several sensors. As an alternative, information may be sent to the system, wired or wirelessly, from a supply system.

What is an acceptable value of the parameter may be preselected, or it may be selected by an operator of the system, or it may be selected by a remote operator. Further, the acceptable value depends on the process. In order to be acceptable, the value should for example be below, or above, a certain limit or within a certain range. If it is above, or below the limit or outside of the range, respectively, the value is unacceptable. Alternatively, there may be a grey-zone, when the system may continue to be used, but when it is recommended to take an action for the system, e.g. change a filter, or to change to another medium supply. The parameters could also be interlinked, meaning that a certain quality level of one parameter may influence an acceptable quality level of one or more other parameters.

By locating the parameter determining unit upstream, i.e. before the device, it is ensured that the parameter of the medium is determined before the medium is supplied to the device. If the medium is continuously supplied, the parameter may be continuously determined, but in any event the parameter of a certain unit of the medium may be determined before that unit is supplied to the device. Thereby, it is possible to take appropriate action, should the medium be unacceptable or questionable, before it reaches the device. It is e.g. possible to adjust the quality of the medium before it enters the device if desirable, and/or needed, e.g. by adding a chemical.

As an alternative, not covered by the claims, or as a complement to the system of claim 1, the parameter of the medium may determined when the medium is being supplied to the device. The parameter may e.g. be determined in the device.

By monitoring the parameter/parameters of the medium, and comparing it with the desired or required quality, it will be possible to assess if the incoming medium is appropriate for the intended use and to take appropriate actions, either automatically by the system, by the operator and/or by the remote operator, in case the medium quality should deviate, before the process or the goods to be washed, disinfected or sterilized by the device is/are significantly affected.

The system further comprises
- a medium input unit, adapted to receive the medium.

The system may further comprise
- a medium supply unit, adapted to supply the medium to the device.

The system may then be configured such that the device and the medium input unit, the parameter determining unit and the medium supply unit are comprised in one common physical unit. Also the device may be comprised in the same physical unit. In that case the medium supply unit may be dispensed with. Alternatively, the optional medium input unit, the parameter determining unit, the optional medium supply unit and/or the device may be separate units.

The system may further comprise at least one display adapted to display an output signal associated with the determined value indicative of the parameter. The display may be a part of the same physical unit as the parameter determining unit or the device, e.g. located in or on the housing of the device. As another alternative, the display may be remotely located. The display may display a value measured by one or several sensors of the parameter determining unit, a rounded-off value or a value presented in a simplified form, e.g. green if acceptable and red if unacceptable. The determined values may e.g. be shown as numbers, as graphs, as line diagrams or as bar diagrams.

If using the above-mentioned grey zone, such values may be presented in yellow. The colour set of green, yellow and red and their meaning are well-known by most people, since these colours are the same as used for traffic signals. The display may be a touch-sensitive display, such that it is able to display information and receive input from the operator. Additionally and/or alternatively, input from the operator or the remote operator may be received through other means, for instance from a keyboard, buttons, knobs and/or wheels associated with the system.

The system may further comprise a memory for storing the determined value indicative of the parameter, preferably for batch-wise storing. Thereby it is possible to perform a follow-up, if it is detected afterwards that an item of medical, dental, laboratory or pharmaceutical goods or a batch has a deviation as regards the hygienic demands after having been processed in the system. The stored data may be used to establish if a certain incoming medium has a lower quality than expected, e.g. resulting in recommendations of e.g. increased service intervals for how often exchange or cleaning of filters should be made. The memory may thus be used for following trends of the parameter of the incoming medium enabling preventive maintenance programs to be launched. Further, if the system should need service, maintenance or repair, the technician may find useful information in the stored data. The technician will be able to detect if there has been any problem or deviation for the incoming medium, which could or will affect the performance of the device. The memory may be a part of the same physical unit as the parameter determining unit or the device, e.g. located in the housing of the device. As another alternative, the memory may be remotely located, or a local memory may be made available to a remote system via a network or similar technology.

The system may further comprise a process stop unit adapted to pause or stop supply of the medium to the device, in case the determined value indicative of the parameter is unacceptable. The process stop unit may be a valve. It may be part of the optional medium supply unit. The process stop unit may be located anywhere upstream of the device. The process stop unit may be connected to the optional display and/or the optional memory, e.g. to show and/or to store if a process stop is performed. There may, in addition, or as a complement, be a process stop unit directly controlling the device. The process stop unit may have different modes, e.g. an immediate stop and a controlled stop, wherein the controlled stop facilitates an easy start-up again of the device. The process stop unit may be used to stop or pause the process.

The system may further comprise a control unit adapted to take an appropriate action to adjust the quality of the medium based on the determined value indicative of the parameter. For example, if pH is determined to be too high an acid may be added, and, if too low, an alkali may be added. The control unit may also be used to control concentration, e.g. if the medium is a detergent. The control unit may be an electrical or an electronical control unit. The control unit may be connected to the optional medium input unit, such that the control unit e.g. can adjust the capacity of a pump. The control unit may be connected to the display, the remote display, the memory and/or the remote memory, e.g. to show and/or to store if it is active or not. The control unit may be controlled by the system, by the operator and/or by the remote operator.

The system may further comprise a communication unit for sending or making the determined value indicative of the parameter available to a remote memory and/or a remote display. The information may be communicated wired- or wirelessly. The remote memory and/or remote display may, as an option, form part of the system. The local display is adapted to display information to an operator or a service technician present at the location of the device, while the remote display may be monitored at another location, e.g. at a monitoring centre, which may be common for many systems, or in a remote position, which is more convenient to the users of the system than on the device itself. Similarly, the local memory is adapted to store information representative of that system, while the remote memory may be common for many systems. The remote memory is located at another location than the device, e.g. at the monitoring centre, or in the remote position, which is more convenient to the users than on the device itself. The remote memory, as well as the local memory, may be used to follow trends for the incoming media.

By using a remote display and/or remote memory the local operator may be relieved from the task of following the parameters. Instead, or as a complement, this task may be performed by the remote operator or by a remote system in the monitoring centre. The remote operator or remote system can then decide, at least partly based on the determined parameters, when it is appropriate to perform service and/or maintenance, e.g. exchange of a filter.

The system may be adapted for supplying a first plurality of media to the device. It may e.g. comprise a first plurality of medium input units. Usually more than one medium is required for the process. Preferably, the system is adapted for handling all, or substantially all, media required for the process.

The system may further comprise a second plurality of parameter determining units, preferably arranged such that a parameter determining unit is arranged for each of most of the incoming media, preferably for substantially all of the incoming media, more preferably for all incoming media. In that case, the first plurality would equal the second plurality. The parameter determining units may be arranged such that each medium has its own parameter determining unit. Alternatively, two or more media may share parameter determining unit, and/or a medium may use more than one parameter determining unit. The parameter of a certain medium is determined before or when the medium is supplied to the device.

The local display and/or the remote display may be adapted to display an output signal associated with the determined value indicative of the parameter of at least one of the first plurality of media, preferably the determined values of at least two of the first plurality of media, and more preferably the determined values of substantially all of the first plurality of media. Even if it would be possible to use a display for an individual medium and/or for an individual parameter, it is preferred to use a common display. More preferably, the display is common for all media, such that an operator of the system only has to monitor one monitor, which may be located on the housing of the device. If the display is remotely located, e.g. at the monitoring centre, the display may be common for many systems. The display may display one or more parameters at the time. The display may change between displaying different views, a view comprising a subset of the determined parameters. Further, if the display comprises a touch-sensitive display or another input device such as a keyboard, knobs or buttons, the operator may interact with the system.

By having the parameter determining unit monitoring the parameters and displaying this information on a common display or a common remote display, it will be possible to asses if the incoming media is appropriate for the intended use before or when being supplied to the device. If a problem occurs, that information can be displayed and communicated before any long term damage or break down of the device occurs or the laboratory or medical goods suffer insufficient processing.

In a second aspect of the present invention, there is provided a process according to claim 10 for supply of medium to a system for washing, disinfecting and/or sterilizing medical, dental, laboratory and/or pharmaceutical goods. The system comprises a device for washing, disinfecting and/or sterilizing medical, dental, laboratory and/or pharmaceutical goods. The process comprises:
- inputting a medium adapted to be used for a process performed in the device,
- determining a value indicative of a parameter of the medium, and
- logging the determined value.

Logging the determined value is herein defined as making a note or a record of the determined value. In practice, the determined value is stored in the local memory and/or the remote memory. Other process data may be logged as well together with the determined value.

The process may be performed for a plurality of media, preferably in parallel.

The process is preferably repeated. It may be performed continuously, or intermittently e.g. once every second, once every minute, once every hour or once every day.

The parameter is a quality parameter, in accordance with the ones mentioned above. The process may further comprise:
- determining if the value is acceptable,
- if acceptable, supplying the medium to the device.

As an extra step, the determined value may be logged, optionally together with process data, for further or future references.

In addition there may be a step of:
- displaying, locally or remotely, an output signal associated with the determined value of the parameter, e.g. at a local or remote display, and/or
- storing, locally or remotely, the determined value indicative of the parameter, e.g. in a local or remote memory.

The process may also comprise a step of:
- sending or making the determined value indicative of the parameter available to a remote system for analysis and/or display, e.g. by means of a communication unit comprised in the system.

Thereby the remote system may analyse and/or display data stored in the device.

The remote system may comprise a memory and/or a display, as mentioned above. The remote system may be monitored at another location, e.g. at a monitoring centre, which may be common for many systems, or in a remote position, which is more convenient to the users than on the device itself.

If the value indicative of the parameter of the medium is unacceptable, one or more of the following steps may be performed:
- displaying information, locally or remotely, that the determined value is unacceptable, and/or
- logging information, locally or remotely, that the determined value is unacceptable, and/or
- triggering an alarm, and/or
- allowing the system to take an appropriate action based on the determined value indicative of the parameter, and/or
- stopping or pausing supply of the medium to the device, and/or
- stopping or pausing the process.

Certain actions may be performed automatically by the system itself, such as increasing capacity of a pump, and/or changing an amount of detergent or solvent per time unit. Other actions may need human interaction, e.g. exchange of a filter or change of medium supply. The operator and/or the remote operator may read information pertaining to when and where to perform this action on the display.

By considering displayed information, retrieved information and/or a warning, the operator or the remote operator may determine that the system should take an appropriate action based on the determined value indicative of the quality parameter. As an alternative, or a complement, the system may automatically perform the appropriate action, e.g. if a certain criterion is fulfilled or certain criteria are fulfilled.

### BRIEF DESCRIPTION OF THE DRAWINGS

The present invention will hereinafter be further explained by means of non-limiting examples with reference to the appended drawings wherein:
- Fig. 1: illustrates a system according to the invention, and
- Fig. 2: illustrates a process according to the invention.

It should be noted that the appended drawings are not necessarily drawn to scale and that the dimensions of some features of the present invention may have been exaggerated for the sake of clarity.

### DETAILED DESCRIPTION

The invention will, in the following, be exemplified by embodiments. It should however be realized that the embodiments are included in order to explain principles of the invention and not to limit the scope of the invention, defined by the appended claims. Details from two or more of the embodiments may be combined with each other.

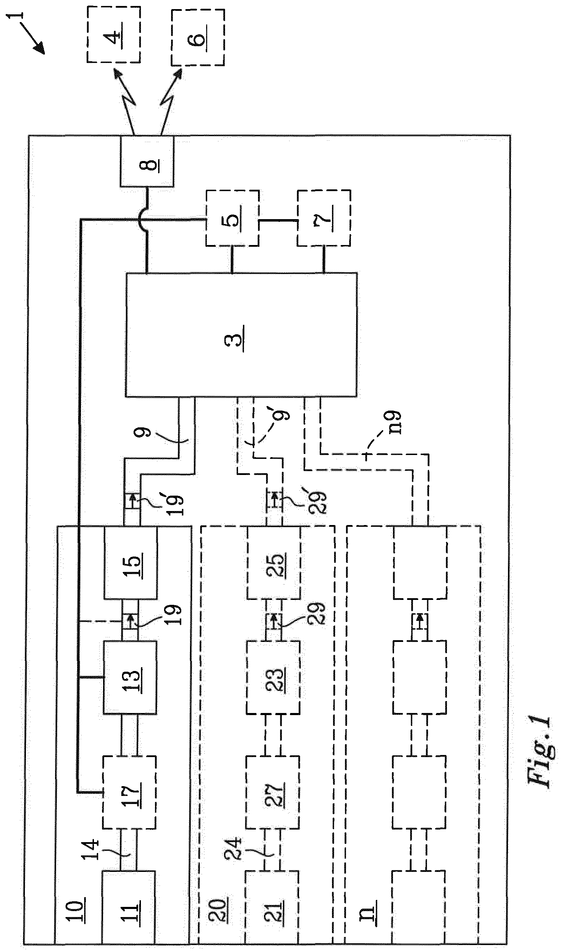

Figure 1 schematically illustrates a system 1 according to the invention. The system 1 comprises a device 3 for washing, disinfecting and/or sterilizing medical, dental, laboratory and/or pharmaceutical goods. The system 1 may further, as an option, comprise a display 5 displaying information about the device 3 and/or an on-going process. The system 1 may also, as an option, comprise a memory 7 for storing information about the device 3 and/or an on-going process. Further, the display 5 may be arranged to display stored information about a previous process, which stored information may be retrieved from the memory 7. The system 1 may further comprise a communication unit 8 for communicating, wired- or wirelessly, information to a remote memory 4 and/or a remote display 6. The remote memory 4 and/or remote display 6 may also, as an option, form part of the system 1. The display 5 is adapted to display information to an operator or a service man present at the device 3, while the remote display 6 may be monitored at another location, e.g. at a monitoring centre, which may be common for a plurality of systems 1. Similarly, the memory 7 is adapted to store information representative of that system 1, while the remote memory 4 may be common for a plurality of systems 1. The remote memory 4 is located at another location than the device 3, e.g. at the monitoring centre. The display 5, the memory 7 and/or the communication unit 8 may be a part of the same physical unit as the device 3, e.g. located in the housing of the device 3.

A feed conduit 9 feeds incoming media to the device 3. Examples of incoming media are water, steam, a solvent, a detergent or a chemical. The media are used for performing the washing, disinfecting and/or sterilizing medical, dental, laboratory and/or pharmaceutical goods process in the device 3.

A first incoming medium is fed through a first subsystem 10. The first medium passes a first medium input unit 11, adapted to receive the medium being supplied to the device 3. The medium input unit 11 may be just an input opening in the system 1, or it may comprise a conduit, a valve and/or a pump. The medium input unit 11 enables the feeding of the medium into the system 1.

The first medium input unit 11 is connected to a first parameter determining unit 13 via a first conduit 14. The first parameter determining unit 13 is adapted to determine a value indicative of a parameter of the first medium, the parameter being a quality parameter. Examples of quality parameters are temperature, pH, conductivity, charge, density and pressure. The first parameter determining unit 13 may be connected to the display 5 or the remote display 6, wired or wirelessly, such that the determined value can be displayed. The displayed value may be a measured value, or it can be presented in a simplified form, e.g. green if acceptable and red if unacceptable. The determined values may e.g. be shown as numbers, as graphs, as line diagrams or bar diagrams. The first parameter determining unit 13 may determine one or more parameters of the first medium. As an alternative, or in addition, there may be more than one parameter determining unit 13 for the first medium determining different parameters of the first medium. The determined value indicative of the parameter of the first medium may be sent to, or displayed by, one or more of the display 5, the memory 7, the remote memory 4 and the remote display 6.

Downstream of the first parameter determining unit 13, there is a first medium supply unit 15 adapted to supply the medium to the device 3. The medium supply unit may be just a conduit leading to the device 3, or it may comprise a valve and/or a pump. The first medium is led through the feed conduit 9 to the device 3.

The first medium input unit 11 and/or the first medium supply unit 15 may be combined into the first parameter determining unit 13, thus together forming the first parameter determining unit 13. The first parameter determining unit 13 is, in the illustrated embodiment, located upstream of the device 3, such that it determines the parameter of the incoming medium, before it reaches the device 3.

In addition to the first medium input unit 11, the first parameter determining unit 13 and the first medium supply unit 15, the first subsystem 10 may further comprise a first control unit 17, adapted to take an appropriate action to adjust the quality of the first medium based on the determined value indicative of the quality parameter. For example, if pH is determined to be too high, an acid may be added, and, if too low, an alkali may be added. The control unit may also be used to control concentration, e.g. if the medium is a detergent. The control unit may be an electrical or an electronical control unit. The control unit 17 may be connected to the medium input unit 11, such that the control unit 17 e.g. can adjust the capacity of a pump. The control unit may be connected to the display 5, the remote display 6, the memory 7 and/or the remote memory 4, e.g. to show and/or to store, whether it is active or not. The first control unit 17 may be controlled by the system 1, by the operator and/or by the remote operator.

The first subsystem 10 may further comprise a first process stop unit 19 adapted to stop or pause supply of the first medium to the device 3, in case the determined value indicative of the quality parameter is unacceptable and/or it cannot be adjusted by means of the first control unit 17. The first process stop unit 19 may be a valve. It may be part of the first medium supply unit 15. Optionally, the first process stop unit may in addition, or instead, be located outside of the first subsystem 10 as denoted by 19', i.e. downstream of the first medium supply unit 15, as long as it is located upstream of the device 3. The first process stop units 19, 19' may be connected to the display 5, the remote display 6, the remote memory 4 and/or the memory 7, e.g. to show and/or to store if a process stop is performed. There may, in addition or as a complement, be a process stop unit, not illustrated, directly controlling the device 3. The process stop unit may have different modes, e.g. an immediate stop and a controlled stop, wherein the controlled stop helps an easy start-up again of the device 3.

Similar to the first subsystem 10, there may be a second subsystem 20 comprising the corresponding units as the first subsystem 10, namely a second medium input unit 21, a second parameter determining unit 23, a second conduit 24 and a second medium supply unit 25, optionally a second control unit 27 and optionally a second process stop unit 29, 29'. The second medium input unit 21 and/or the second medium supply unit 25 may be combined into the second parameter determining unit 23, thus forming the second parameter determining unit 23. These units 21, 23, 24, 25, 27, 29, 29' have similar functions as already described for the first subsystem 10. The second medium is led through the conduit 9' to the device 3.

There may be a plurality of n subsystems, similar to the first subsystem 10, for example one for each incoming medium. There may be a first plurality of incoming media, each of which may have a corresponding medium input unit 11, 21,... . There may be a second plurality of parameter determining units 13, 23,.... . The parameter determining units may be arranged such that each medium has its own parameter determining unit 13, 23,.... . Alternatively, two or more media may share a parameter determining unit, and/or a medium may use more than one parameter determining unit.

One or more of the subsystems 10, 20 may be a part of the same physical unit as the device 3, e.g. located in the housing of the device 3.

Even though it would be possible to use a display for each medium and/or for each parameter, it is preferred to use a common display 5 or a common remote display 6. More preferably, the display is common for all media, such that an operator of the system 1 only has to monitor one monitor, i.e. the display 5, which may be located on the housing of the device 3, or such that the remote display 6 will be able to display parameters for most, or preferably all, of the incoming media.

By having each subsystem 10, 20 monitoring its parameters and displaying this information on the common display 5 and/or the remote display 6, it will be possible to asses if the incoming media is appropriate for the intended use, before it is supplied to the device 3. Further, if a problem occurs, that information can be displayed and communicated before any long term damage or break down occurs or medical, dental, laboratory and/or pharmaceutical goods suffer insufficient processing.

Purely as an example, the system 1 may communicate via the display 5 and/or the remote display 6 a long time ahead the fact that a filter for an incoming medium, e.g. water is due to be exchanged within a certain time interval. As the time of change is approaching, the communication unit 8 may transfer a request to change the filter, and finally if the filter has not been changed, a warning may be displayed that the device 3 will no longer work properly.

As an alternative, not covered by the claims, or a complement to the system described herein, however not illustrated, the parameter of the medium may be determined when the medium is supplied to the device. The parameter may e.g. be determined in the device 3.

The determined values indicative of the quality parameters may be stored in the memory 7 and/or the remote memory 4. The determined values may be stored together with time data relating to when they were determined. The determined values may further be stored batch-wise, such that it is possible to perform a follow-up, if it is subsequently detected that an item of medical, dental, laboratory or pharmaceutical goods or a batch has a deviation as regards the hygienic demands after having been processed in the system 1. The stored data may be used to see if a certain incoming medium has a lower quality than expected, e.g. resulting in recommendations of for example increased service intervals for how often exchange or cleaning of filters should be made. Further, if the system 1 should need service, maintenance or repair, the technician may find useful information in the stored data. The technician will be able to detect if there has been any problem or deviation for an incoming medium, which could or will affect the performance of the device. Preferably, data for all incoming media are stored in a common central memory 7 and/or the remote memory 4, even if it would also be possible to use memories located in the subsystem 10, 20. The stored data may also be used to determine when it is appropriate to call a technician for service or maintenance of the system 1, the device 3 or of any of its subsystems 10, 20... This may be done automatically if a certain criterion is fulfilled or certain criteria are fulfilled. Alternatively, or as a complement, the operator and/or the remote operator may decide when to call a technician for service or maintenance.

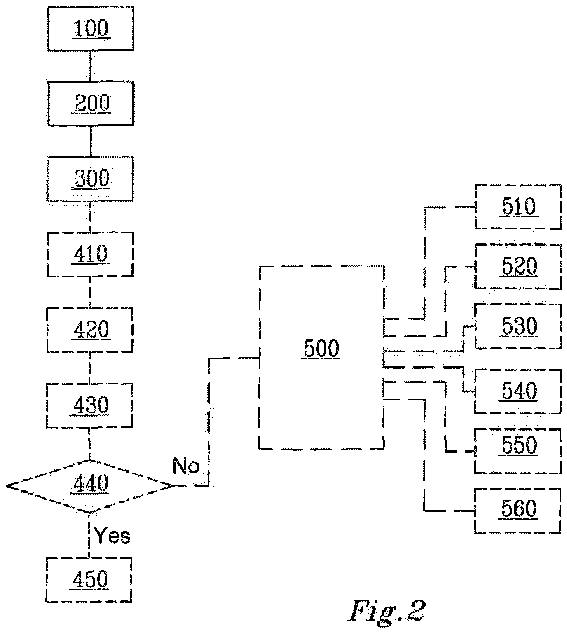

Figure 2 illustrates a process for supply of a medium to a system 1 for washing, disinfecting and/or sterilizing medical, dental, laboratory and/or pharmaceutical goods according to a second aspect of the present invention. The system 1 comprises a device 3 for washing, disinfecting and/or sterilizing medical, dental, laboratory and/or pharmaceutical goods. The reference numbers used for the system 1 refer to Figure 1, while the reference numbers of the process refer to Figure 2.

In its simplest form the process comprises:
100: Inputting a medium adapted to be used for a process performed in the device 3.
200: Determining a value indicative of a parameter of the medium.
300: Logging the determined value.

The parameter is a quality parameter, as described above.

The process may be performed for a plurality of media in parallel.

The process is preferably repeated. It may be performed continuously, or intermittently e.g. once every second.

The process may further comprise:
410: Sending or making the determined value indicative of the parameter available to a remote system for analysis and/or display, e.g. by means of the communication unit 8.

The process may further comprise
420: Displaying, locally and/or remotely, an output signal associated with the determined value indicative of the parameter.

The display may be the local display 5 and/or the remote display 6.

The process may further comprise:
430: Storing, locally and/or remotely, the determined value indicative of the parameter.

The determined value may be stored in the memory 7 and/or the remote memory 4.

In addition the process may comprise the steps of:
440: Determining if the value is acceptable,
450: If acceptable, supplying the medium to the device 3.

The determined value may be logged, optionally together with process data, for further or future references.

The process may comprise one or more of the optional steps 410, 420, 430, or 440+450.

If the value indicative of the quality parameter of the medium is unacceptable, step 500, one or more of the following steps may be performed:
510: Displaying information, locally and/or remotely, that the determined value is unacceptable.
520: Logging information, locally and/or remotely, that the determined value is unacceptable.
530: Triggering an alarm that the value is unacceptable. The alarm signal may be sent as a visual, acoustic and/or haptic alarm signal.
540: Allowing the system 1 to take an appropriate action based on the determined value indicative of the quality parameter.
550: Stopping or pausing supply of the medium to the device 3.
560: Stopping or pausing the process of washing, disinfecting and/or sterilizing.

Certain actions, such as the ones of step 540 may be performed by the system 1 itself, possibly automatically, such as increasing capacity of a pump, changing an amount of detergent or solvent. Other actions may need human interaction, e.g. exchange of a filter or change of a medium supply. Information about when and where to perform this action may be read on the display 5 or the remote display 6.

Considering displayed information, retrieved information and/or a warning, the operator or the remote operator may determine that the system 1 should take an appropriate action based on the determined value indicative of the quality parameter. As an alternative, or a complement, the system 1 may automatically perform the appropriate action, e.g. if a certain criterion is fulfilled or certain criteria are fulfilled.

Further modifications of the invention within the scope of the appended claims are feasible. As such, the present invention should not be considered as limited by the embodiments and figures described herein. Rather, the full scope of the invention should be determined by the appended claims.

## Claims

1. A system (1) comprising
- a device (3) for washing, disinfecting and/or sterilizing medical, dental, laboratory and/or pharmaceutical goods, and
- a parameter determining unit (13, 23) adapted to determine a value indicative of a parameter of a medium dedicated to the device before being supplied to the device (3),
wherein the parameter is representative of a quality aspect of the medium, said parameter being taken from the list of temperature, pH, conductivity, density, pressure, and wherein the medium supplied to the device is used for a process performed in the device, said medium being a liquid, a gas or an aerosol,
the system further comprising a medium input unit (11, 21) adapted to receive the medium, which medium input unit (11, 21) enables feeding of the medium into the system, and which medium input unit (11, 21) is connected to the parameter determining unit (13, 23) by a conduit (14),
wherein the parameter determining unit (13, 23) is located upstream of the device (3), such that it determines the parameter of the incoming medium, before it reaches the device (3) and such that only the quantity of the medium which is intended to be supplied to the device (3) is monitored by the parameter determining unit (13, 23).

2. The system (1) according to claim 1, wherein said medium is water, steam, a solvent or a detergent.

3. The system (1) according to any one of the preceding claims further comprising a memory (7) for storing said determined value indicative of said parameter, preferably for batch-wise storing.

4. The system (1) according to any one of the preceding claims further comprising a control unit (17, 27) adapted to take an appropriate action to adjust the quality of said medium based on said determined value indicative of said parameter.

5. The system (1) according to any one of the preceding claims further comprising a communication unit (8) for sending or making said determined value indicative of said parameter available to a remote memory (4) and/or a remote display (6).

6. The system (1) according to any one of the preceding claims being adapted for supplying a first plurality of media to said device (3).

7. The system (1) according to claim 6, further comprising a second plurality of parameter determining units (13, 23), preferably arranged such that a parameter determining unit (13; 23) is arranged for each of said first plurality of media.

8. The system (1) according to claim 6 or 7, comprising a display (5) and/or a remote display (6), wherein said display (5) and/or remote display (6) is adapted to display an output signal associated with said determined value indicative of said parameter of at least one of said first plurality of media, preferably said determined values of at least two of said first plurality of media and more preferably said determined values of all of said first plurality of media.

9. The system (1) according to any one of the preceding claims wherein said medium input unit (11, 21), said parameter determining unit (13, 23) and said device (3) are comprised in one common physical unit.

10. A process for supply of a medium to a system (1) for washing, disinfecting and/or sterilizing medical, dental, laboratory and/or pharmaceutical goods, said system (1) comprising a device (3) for washing, disinfecting and/or sterilizing medical, dental, laboratory and/or pharmaceutical goods, said process comprising
100: inputting a medium adapted to be used for a process performed in said device (3) by means of a medium input unit (11), thereby enabling feeding of said medium into said system, said medium being a liquid, a gas or an aerosol,
200: determining a value indicative of a parameter of said medium before said medium is supplied to said device (3) by means of a parameter determining unit (13, 23), thereby monitoring only the quantity of said medium which is intended to be supplied to said device, wherein said parameter is representative of a quality aspect of said medium, said parameter being taken from the list of temperature, pH, conductivity, density, pressure, and
300: logging said determined value.

11. The process according to claim 10 being repeated, preferably being performed intermittently or continuously.

12. The process according to any one of claims 10 to 11 further comprising
440: determining if said value is acceptable,
450: if acceptable, supplying said medium to said device (3).

13. The process according to any one of claims 10 to 12 further comprising
500: if said value is unacceptable,
510: displaying information, locally and/or remotely that said determined value is unacceptable, and/or
520: logging information, locally and/or remotely, that said determined value is unacceptable, and/or
530: triggering an alarm, and/or
540: allowing said system (1) to take an appropriate action based on said determined value indicative of said parameter, and/or
550: stopping or pausing supply of said medium to said device (3), and/or
560: stopping or pausing said process.

## Patentansprüche

1. System (1), umfassend
- eine Vorrichtung (3) zum Waschen, Desinfizieren und/oder Sterilisieren von medizinischen, zahnmedizinischen, labortechnischen und/oder pharmazeutischen Gütern und
- eine Parameterbestimmungseinheit (13, 23), die dazu ausgelegt ist, einen Wert zu bestimmen, der einen Parameter eines Mediums angibt, das für die Vorrichtung vorgesehen ist, bevor es der Vorrichtung (3) zugeführt wird,
wobei der Parameter repräsentativ für einen Qualitätsaspekt des Mediums ist, wobei der Parameter aus der Liste aus Temperatur, pH-Wert, Leitfähigkeit, Dichte, Druck entnommen ist und wobei das der Vorrichtung zugeführte Medium für einen in der Vorrichtung durchgeführten Prozess verwendet wird, wobei das Medium eine Flüssigkeit, ein Gas oder ein Aerosol ist,
wobei das System ferner eine Mediumeingabeeinheit (11, 21) umfasst, die dazu ausgelegt ist, das Medium aufzunehmen, wobei die Mediumeingabeeinheit (11, 21) ein Einspeisen des Mediums in das System ermöglicht und wobei die Mediumeingabeeinheit (11, 21) mit der Parameterbestimmungseinheit (13, 23) durch eine Leitung (14) verbunden ist,
wobei die Parameterbestimmungseinheit (13, 23) stromaufwärts der Vorrichtung (3) angeordnet ist, sodass sie den Parameter des einströmenden Mediums bestimmt, bevor es die Vorrichtung (3) erreicht, und sodass nur die Menge des Mediums, die der Vorrichtung (3) zugeführt werden soll, durch die Parameterbestimmungseinheit (13, 23) überwacht wird.

2. System (1) nach Anspruch 1, wobei das Medium Wasser, Dampf, ein Lösungsmittel oder ein Detergens ist.

3. System (1) nach einem der vorhergehenden Ansprüche, ferner umfassend einen Speicher (7) zum Speichern des bestimmten Werts, der den Parameter angibt, bevorzugt zum stapelweisen Speichern.

4. System (1) nach einem der vorhergehenden Ansprüche, ferner umfassend eine Steuereinheit (17, 27), die dazu ausgelegt ist, eine zweckmäßige Maßnahme zum Einstellen der Qualität des Mediums auf Grundlage des bestimmten Werts, der den Parameter angibt, zu ergreifen.

5. System (1) nach einem der vorhergehenden Ansprüche, ferner umfassend eine Kommunikationseinheit (8) zum Senden oder Verfügbarmachen des bestimmten Werts, der den Parameter angibt, an einen entfernten Speicher (4) und/oder eine entfernte Anzeige (6) .

6. System (1) nach einem der vorhergehenden Ansprüche, das zum Zuführen einer ersten Vielzahl von Medien zu der Vorrichtung (3) ausgelegt ist.

7. System (1) nach Anspruch 6, ferner umfassend eine zweite Vielzahl von Parameterbestimmungseinheiten (13, 23), die bevorzugt derart angeordnet ist, dass eine Parameterbestimmungseinheit (13; 23) für jedes der ersten Vielzahl von Medien angeordnet ist.

8. System (1) nach Anspruch 6 oder 7, umfassend eine Anzeige (5) und/oder eine entfernte Anzeige (6), wobei die Anzeige (5) und/oder die entfernte Anzeige (6) dazu ausgelegt ist, ein Ausgangssignal anzuzeigen, das mit dem bestimmten Wert assoziiert ist, der den Parameter von mindestens einem der ersten Vielzahl von Medien angibt, bevorzugt die bestimmten Werte von mindestens zwei der ersten Vielzahl von Medien und weiter bevorzugt die bestimmten Werte von allen der ersten Vielzahl von Medien.

9. System (1) nach einem der vorhergehenden Ansprüche, wobei die Mediumeingabeeinheit (11, 21), die Parameterbestimmungseinheit (13, 23) und die Vorrichtung (3) in einer gemeinsamen physischen Einheit umfasst sind.

10. Prozess zur Zufuhr eines Mediums zu einem System (1) zum Waschen, Desinfizieren und/oder Sterilisieren von medizinischen, zahnmedizinischen, labortechnischen und/oder pharmazeutischen Gütern, wobei das System (1) eine Vorrichtung (3) zum Waschen, Desinfizieren und/oder Sterilisieren von medizinischen, zahnmedizinischen, labortechnischen und/oder pharmazeutischen Gütern umfasst, wobei der Prozess Folgendes umfasst:
100: Eingeben eines Mediums, das dazu ausgelegt ist, für einen in der Vorrichtung (3) durchgeführten Prozess verwendet zu werden, mittels einer Mediumeingabeeinheit (11), wodurch ein Einspeisen des Mediums in das System ermöglicht wird, wobei das Medium eine Flüssigkeit, ein Gas oder ein Aerosol ist,
200: Bestimmen eines Werts, der einen Parameter des Mediums angibt, bevor das Medium der Vorrichtung (3) zugeführt wird, mittels einer Parameterbestimmungseinheit (13, 23), wodurch nur die Menge des Mediums, die der Vorrichtung zugeführt werden soll, überwacht wird, wobei der Parameter repräsentativ für einen Qualitätsaspekt des Mediums ist, wobei der Parameter aus der Liste aus Temperatur, pH-Wert, Leitfähigkeit, Dichte, Druck entnommen ist, und
300: Protokollieren des bestimmten Werts.

11. Prozess nach Anspruch 10, der wiederholt wird, bevorzugt intermittierend oder kontinuierlich durchgeführt wird.

12. Prozess nach einem der Ansprüche 10 bis 11, ferner umfassend
440: Bestimmen, ob der Wert akzeptabel ist,
450: falls akzeptabel, Zuführen des Mediums zu der Vorrichtung (3) .

13. Prozess nach einem der Ansprüche 10 bis 12, ferner umfassend 500: falls der Wert inakzeptabel ist,
510: Anzeigen von Informationen, lokal und/oder entfernt, dass der bestimmte Wert inakzeptabel ist, und/oder
520: Protokollieren von Informationen, lokal und/oder entfernt, dass der bestimmte Wert inakzeptabel ist, und/oder
530: Auslösen eines Alarms und/oder
540: Ermöglichen, dass das System (1) auf Grundlage des bestimmten Werts, der den Parameter angibt, eine zweckmäßige Maßnahme ergreift, und/oder
550: Anhalten oder Pausieren der Zufuhr des Mediums zu der Vorrichtung (3), und/oder
560: Anhalten oder Pausieren des Prozesses.

## Revendications

1. Système (1) comprenant
- un dispositif (3) de lavage, de désinfection et/ou de stérilisation de produits médicaux, dentaires, de laboratoire et/ou pharmaceutiques, et
- une unité de détermination de paramètre (13, 23) conçue pour déterminer une valeur indicative d'un paramètre d'un milieu dédié au dispositif avant d'être fourni au dispositif (3),
dans lequel le paramètre est représentatif d'un aspect de qualité du milieu, ledit paramètre étant pris dans la liste de température, pH, conductivité, densité, pression, et dans lequel le milieu fourni au dispositif est utilisé pour un processus exécuté dans le dispositif, ledit milieu étant un liquide, un gaz ou un aérosol,
le système comprenant en outre une unité d'entrée de milieu (11, 21) conçue pour recevoir le milieu, laquelle unité d'entrée de milieu (11, 21) permet l'introduction du milieu dans le système, et laquelle unité d'entrée de milieu (11, 21) est connectée à l'unité de détermination de paramètre (13, 23) par un conduit (14),
dans lequel l'unité de détermination de paramètre (13, 23) est située en amont du dispositif (3), de sorte qu'elle détermine le paramètre du milieu entrant, avant qu'il n'atteigne le dispositif (3) et de sorte que seulement la quantité du milieu qui est destinée à être fournie au dispositif (3) est surveillée par l'unité de détermination de paramètre (13, 23).

2. Système (1) selon la revendication 1, dans lequel ledit milieu est de l'eau, de la vapeur, un solvant ou un détergent.

3. Système (1) selon l'une quelconque des revendications précédentes comprenant en outre une mémoire (7) pour stocker ladite valeur déterminée indicative dudit paramètre, de préférence pour un stockage par lots.

4. Système (1) selon l'une quelconque des revendications précédentes comprenant en outre une unité de commande (17, 27) conçue pour effectuer une action appropriée pour régler la qualité dudit milieu en fonction de ladite valeur déterminée indicative dudit paramètre.

5. Système (1) selon l'une quelconque des revendications précédentes comprenant en outre une unité de communication (8) pour envoyer ou rendre ladite valeur déterminée indicative dudit paramètre disponible à une mémoire distante (4) et/ou un affichage distant (6).

6. Système (1) selon l'une quelconque des revendications précédentes étant conçu pour fournir une première pluralité de milieux audit dispositif (3).

7. Système (1) selon la revendication 6, comprenant en outre une seconde pluralité d'unités de détermination de paramètre (13, 23), de préférence agencées de sorte qu'une unité de détermination de paramètre (13 ; 23) est agencée pour chacun de ladite première pluralité de milieux.

8. Système (1) selon la revendication 6 ou 7, comprenant un affichage (5) et/ou un
affichage à distance (6), dans lequel ledit affichage (5) et/ou affichage à distance (6) est conçu pour afficher un signal de sortie associé à ladite valeur déterminée indicative dudit paramètre d'au moins l'un de ladite première pluralité de milieux, de préférence lesdites valeurs déterminées d'au moins deux de ladite première pluralité de milieux et plus préférablement lesdites valeurs déterminées de l'ensemble de ladite première pluralité de milieux.

9. Système (1) selon l'une quelconque des revendications précédentes, dans lequel ladite unité d'entrée de milieu (11, 21), ladite unité de détermination de paramètre (13, 23) et ledit dispositif (3) sont compris dans une unité physique commune.

10. Processus de fourniture d'un milieu à un système (1) pour laver, désinfecter et/ou stériliser des produits médicaux, dentaires, de laboratoire et/ou pharmaceutiques, ledit système (1) comprenant un dispositif (3) pour laver, désinfecter et/ou stériliser des produits médicaux, dentaires, de laboratoire et/ou pharmaceutiques, ledit processus
comprenant :
100 : entrée d'un milieu conçu pour être utilisé pour un processus exécuté dans ledit dispositif (3) au moyen d'une unité d'entrée de milieu (11), permettant ainsi l'introduction dudit milieu dans ledit système, ledit milieu étant un liquide, un gaz ou un aérosol,
200 : détermination d'une valeur indicative d'un paramètre dudit milieu avant que ledit milieu ne soit fourni audit dispositif (3) au moyen d'une unité de détermination de paramètre (13, 23), surveillant ainsi uniquement la quantité dudit milieu qui est destinée à être fournie audit dispositif, dans lequel ledit paramètre est représentatif d'un aspect de qualité dudit milieu, ledit paramètre étant pris dans la liste de température, pH, conductivité, densité, pression, et
300 : enregistrement de ladite valeur déterminée.

11. Processus selon la revendication 10 étant répété, de préférence étant exécuté par intermittence ou en continu.

12. Processus selon l'une quelconque des revendications 10 à 11 comprenant en outre
440 : le fait de déterminer si ladite valeur est acceptable
450 : si acceptable, fourniture dudit milieu audit dispositif (3) .

13. Processus selon l'une quelconque des revendications 10 à 12 comprenant en outre
500 : si ladite valeur est inacceptable,
510 : affichage d'informations, localement et/ou à distance, selon lesquelles ladite valeur déterminée est inacceptable, et/ou
520 : enregistrement d'informations, localement et/ou à distance, selon lesquelles ladite valeur déterminée est inacceptable, et/ou
530 : déclenchement d'une alarme, et/ou
540 : autorisation dudit système (1) à effectuer une action appropriée sur la base de ladite valeur déterminée indicative dudit paramètre, et/ou
550 : arrêt ou mise en pause de la fourniture dudit milieu audit dispositif (3), et/ou
560 : arrêt ou mise en pause dudit processus.
